Europäisches Patentamt

European Patent Office

Office européen des brevets

(11) Publication number: **0 097 934**
**B1**

# EUROPEAN PATENT SPECIFICATION

(45) Date of publication of patent specification: **28.02.90**

(21) Application number: **83106199.9**

(22) Date of filing: **24.06.83**

(51) Int. Cl.⁵: **A 61 B 1/00,** A 61 B 1/12,
B 29 C 47/06, G 02 B 23/26

(54) A composite optical fiber and imaging catheter and method for producing the same.

(30) Priority: **26.06.82 JP 110151/82**

(43) Date of publication of application:
**11.01.84 Bulletin 84/02**

(45) Publication of the grant of the patent:
**28.02.90 Bulletin 90/09**

(84) Designated Contracting States:
**DE FR GB IT NL**

(56) References cited:
**DE-A-3 014 407**
**DE-B-13 028 09**
**GB-A-2 108 391**
**US-A-2 843 112**
**US-A-3 089 484**
**US-A-3 554 721**
**US-A-3 641 332**
**US-A-4 125 644**

**TRANSACTIONS OF THE IECE OF JAPAN,
vol.E-61, no. 3, March 1978, pages 175-177,
Tokyo, JP; SUZUKI et al.: "Flat type cable with
silicone clad optical fibers"**

(73) Proprietor: **SUMITOMO ELECTRIC INDUSTRIES
LIMITED**
**No. 15, Kitahama 5-chome Higashi-ku**
**Osaka-shi Osaka 541 (JP)**

(72) Inventor: **Yoshida, Kenichi c/o Osaka Works of
Sumitomo**
**Electric Ind. Ltd. No. 1-3, Shimaya 1-chome
Konohana-ku Osaka-shi Osaka (JP)**
Inventor: **Ono, Kimizo c/o Osaka Works of
Sumitomo**
**Electric Ind. Ltd. No. 1-3, Shimaya 1-chome
Konohana-ku Osaka-shi Osaka (JP)**
Inventor: **Tsuno, Koichi c/o Osaka Works of
Sumitomo**
**Electric Ind. Ltd. No. 1-3, Shimaya 1-chome
Konohana-ku Osaka-shi Osaka (JP)**

(74) Representative: **Patentanwälte Grünecker,
Kinkeldey, Stockmair & Partner**
**Maximilianstrasse 58**
**D-8000 München 22 (DE)**

(56) References cited:
**Electronics Letters,Vol. 11, Nr 7, April 1975,
Pages 153-154**

**Werkstoff,Führer Kunstoffe, Pages 274-275**

## Description

The present invention relates to a method of making an imaging catheter, and to an imaging catheter made according to such method.

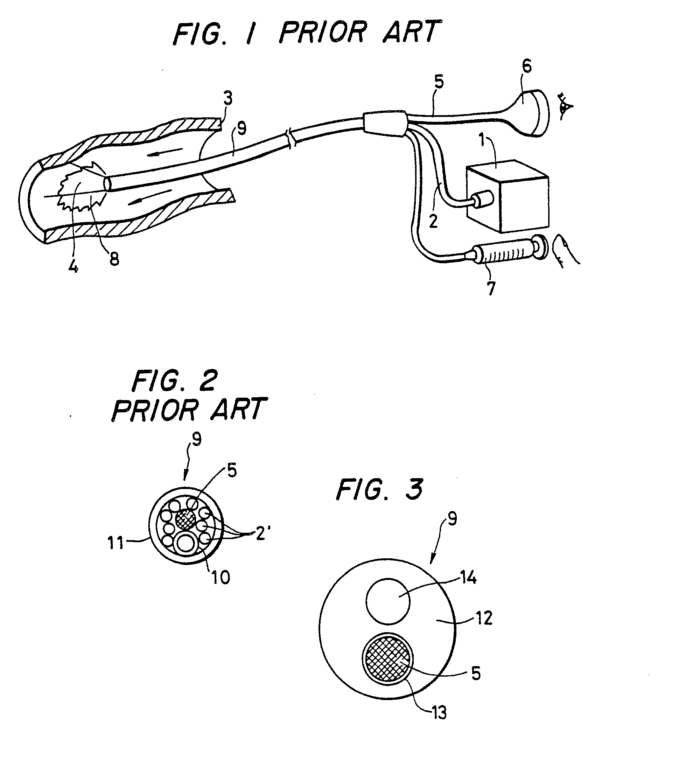

An endoscope (hereinafter referred to as an "imaging catheter") is conventionally used to examine blood vessels, the heart, and the interior of body cavities. A typical imaging catheter is shown in Figure 1 wherein an illuminating light 4 from a light source 1 is guided through an optical transmission fiber 2 into a blood vessel 3. The image of the target, which is formed by a lens installed at the tip of the catheter, is sent back through an image transmitting optical fibre bundle 5 to a direct-vision adapter 6 which enlarges the image for direct viewing by an operator. If the target is the wall of the blood vessel, and its viewing is obstructed by the blood flowing between the catheter and the target, a physiological saline solution 8 is supplied from a syringe 7 and squirted from the top of the catheter to flush away the blood.

As shown in Figure 2, and known composite opitcal fiber unit 9, which makes up the imaging catheter that achieves these functions, comprises the fiber bundle 5, a brine transfering tube 10 and as a transparent transmitting structure a plurality of small diameter optical fibers 2' for transmitting the illuminating light, and all of these elements are confined in a casing 11 such as a heat-shrinkable tube. However, since the optical fibers 2' are circular in cross-section, a gap exists between them and also between the fiber bundle 5 and the brine transfer tube 10. As a result, the cross-sectional area, through which the illuminating light is transmitted, is relatively small with respect to the overall cross-section of the imaging catheter. Furthermore, the casing or sheath 11 that is used to retain the optical fibers 2' around the fiber bundle 5 unavoidably adds to the outside diameter of the fiber unit 9. As a further disadvantage, assembling and arranging a plurality of optical illumination transfer fibers 2', a fiber bundle 5 and a brine transfer tube 10 requires considerable time and labour, and, consequently, the cost of producing the composite optical fiber unit 9 is increased.

US—PS 32 089 484 describes a flexible optical instrument comprising a composite optical fiber unit which utilizes a plurality of thin optical fibers for both the transparent illuminating light-transmitting structure 27 and the imaging optical fiber bundle 26, the latter having a relatively large diameter as compared to the former. The imaging optical fiber bundle is surrounded by an opaque thin walled member. This known composite optical fiber unit is manufactured by forming the illuminating light transmitting structure separately from the imaging optical fiber bundle on a thin opaque sheet extending about the interior of the hollow portion of the illuminating light transmitting structure into which the imaging optical fiber bundle is then inserted.

DE—AS 1 302 809 describes in endoscope having a composite optical fiber unit which utilizes a number of thin optical fibers for both the illuminating light transmitting structure and the imaging optical fiber bundle contained therein.

US—PS 2 843 112 describes an endoscope comprising a composite optical member which includes a tapered cylinder 20 of transparent material and as an imaging optical member a transparent rod 22 contained therein. A barrier 27 is provided between both members, to prevent transmission of light therein.

GB—A 2 108 391 describes a speculum for medical instruments comprising a tube-like member made of a light conducting plastic material which carries a light reflecting coating along its inside wall.

A primary object of the present invention is to provide an easy and low cost method of making an imaging catheter, as described at the beginning, having a fine composite optical fiber unit which can be produced efficiently and continuously and which comprises a light transmitting structure having an increased cross-sectional area with respect to the overall outside diameter of the composite optical fiber unit.

This object is achieved by the features of the claims 1 and 5.

Further embodiments are claimed in the subclaims.

One way of carrying out the invention is described in detail below with reference to the drawings which illustrate one embodiment and in which:

Figure 1 is a diagrammatic view of a conventional imaging catheter;

Figure 2 is a cross section of a conventional composite optical fiber unit for use with the imaging catheter of Figure 1;

Figure 3 is a cross section showing a composite optical unit for use with an imaging catheter of the present invention;

Figure 4 is a side, elevational cross section of an apparatus for fabricating the composite fiber unit as shown in Fig. 3;

Figure 5 is a cross section of Figure 4 as taken along line V—V;

Figure 6 diagrammatically shows the structure of a branching mount for an imaging catheter of the present invention;

Figure 7 diagrammatically shows the structure of another branching mount that can be used with the imaging catheter of the present invention;

Figure 8 diagrammatically shows an imaging catheter of the present invention which is used for dental purposes; and

Figure 9 is a cross section of the composite optical fiber unit used with the dental imaging catheter of Figure 8.

Preferred embodiments of the present invention are described by reference to Figures 3—9.

Figure 3 is a cross section of one embodiment of a composite optical fiber unit 9 used for the imagery catheter of the present invention. The composite optical fiber unit 9 comprises an image-transmitting optical fiber bundle 5 which receives information-carrying light from a target; an elongate body of transparent material 12 that includes said fiber bundle 5 in its interior and which transmits an illuminating light from a light source to the target; a light-absorbing layer 13 which is formed around the fiber bundle 5; and a longitudinal opening 14 which is cut through the elongate body of transparent material 12, parallel to the fiber bundle 5, to serve as a brine conduit for permitting the flow of a physiological saline solution.

The fiber bundle 5 is prepared by arranging a plurality of optical fibers in a quartz tube, in a side-by-side assembly, and drawing the assembly to form finer filaments. The light-absorbing layer 13 prevents the leakage of light from the fiber bundle 5 and is made of a material that has a low light transmittance and a higher refractive index than quartz. A suitable example is a hard silicone resin mixed with a fine carbon powder. The elongate body 12 is made of a material which has a high light transmittance such a polymethyl methacrylate, polystyrene or polycarbonate, and it may contain a cladding to eliminate surface flaws, dirt or other factors which may reduce the transmission efficiency of the illuminating light. A suitable cladding may be formed by coating the outer surface of the elongate body 12 and the inner surface of the longitudinal opening 14 with a plastic which has a lower index of refraction than the transparent material. The cladding on the elongate body 12 may be further coated with a fluorinated resin, such as Teflon®, to provided better slip and more effective protection from surface flaws and dirt.

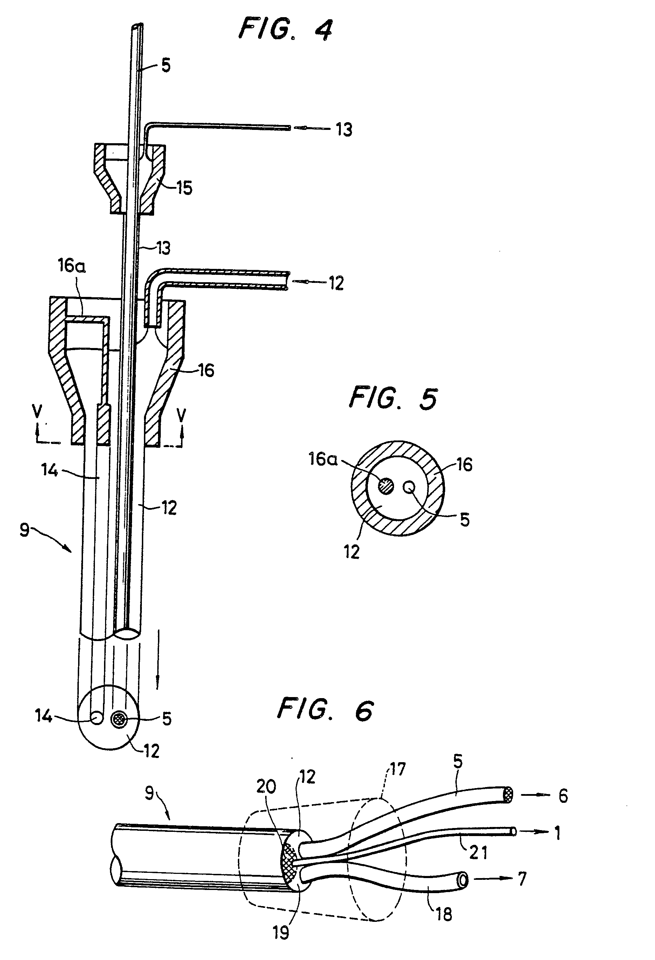

The fiber bundle 5 is formed as an integral part of the plastic elongate body of transparent materials 12, and this can be achieved by extrusion using an apparatus of the type illustrated in Figures 4 and 5. A the fiber bundle 5 is passed through a first die 15, it is coated with a light-absorbing layer 13 which is extruded from the die 15. Then, the fiber bundle 5, with the light-absorbing layer 13 coated onto it, is passed through a second lower die 16 to form an elongate body of transparent plastic material 12. At the same time, a longitudinal opening 14, which forms the passage for the physiological saline solution, is formed in the body 12 by means of an element 16a which is installed in the die 16. The resulting composite optical fiber unit 9 emerges from the bottom of the die 16 and is continuously wound on a capstan take-up roller (not shown) in the direction indicated by the arrow.

An imaging catheter is prepared from the above composite optical fiber unit 9 by the following method. As shown in Figure 6, an end portion of the composite optical fiber unit 9, which is to be equipped with a branching mount 17, is treated with sulfuric acid or the like to remove the body of transparent material 12 and leave only the image fiber bundle 5 behind. A tube 18, which is connected to a syringe (not shown), is inserted into the exposed end of the brine conduit 14 and fixed by a suitable adhesive. Part of the exposed end-surface 19 of the body 12 is a mirror-polished to provide a mirror-smooth surface 20, and one end of an auxiliary optical fiber 21 for transmitting illuminating light, whose other end is connected to a light source (not shown), is attached to the mirror-smooth surface 20 with a film of matching oil which is spread on that surface. The illuminating light from the light source is then fed to the body of transparent plastic material 12 after is has been guided through this auxiliary optical fiber 21. The downstream end of the fiber bundle 5 is connected to a direct-vision adapter (not shown), and the branching section of the optical fiber unit 9 is covered with the branching mount 17 to protect the joint.

Figure 17 shows the structure of another branching mount 17 that can be used with the imaging catheter of the present invention. In this embodiment, the part of the optical fiber unit which is to be equipped with the branching mount 17 and an end portion of the optical fiber are split into two portions in the longitudinal direction, and the fiber bundle 5 is then separated from the body of transparent material 12. As in the embodiment shown in Figure 6, a tube 18 is inserted into the exposed end of the brine conduit 14 and fixed by an adhesive. The split portions of the body of transparent material 12 which extend from the branching mount 17 are joined by heating or another suitable means, and the downstream end of the so-joined transparent material 12 is connected to the light source (not shown).

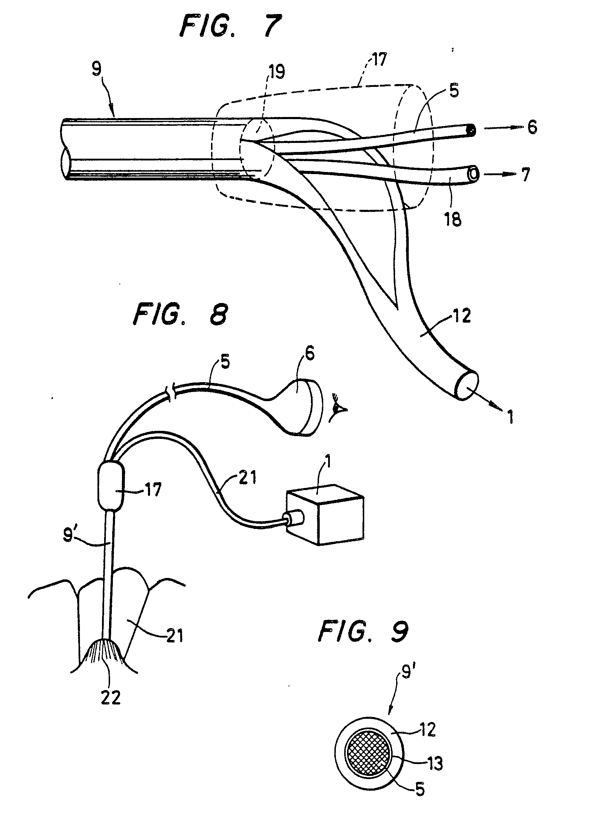

Figures 8 and 9 show an embodiment in which the optical fiber unit of the present invention is used as a dental fiber unit for examining the interior of a tooth 21 or the gingiva 22. In practice, a small hole is cut down to the gingiva 22 in the tooth 21, and the tip of the optical fiber unit 9', including the light-illuminating fiber and fiber bundle, is inserted into that hole. The outside diameter of the optical fiber unit 9', when used as a dental imaging fiber unit, must not exceed 0.7 mm, and this has been impossible when the conventional fiber arrangement has been used. However, the optical fiber unit 9' of the present invention satisifies this rigorous dimensional requirement by forming the transparent, light-transmitting body of plastic material 12 concentric around, and as an integral part of, the imaging fiber bundle 5 with the light-absorbing layer 13, as shown in Figure 9.

The composite optical fiber unit of the present invention can be used as an imaging catheter with an endoscope for examining blood vessels or the heart, and it can also be used as an imaging catheter with an endoscope in dentistry, ophthalmology, otolaryngology or urology.

As described above, the composite fiber unit of the present invention does not have a gap formed between the transparent, light-illuminating material, the imaging optical fiber bundle, or the brine conduit; therefore, it has an increased cross-sectional area for transmitting the illuminating light with respect to the overall outside diameter of the optical fiber unit. Moreoover, the

optical fiber unit of the present invention can be continuously and efficiently produced by the extrusion technique of light-illuminating fibers and encasing them with a heat-shrinkable tube. As a further advantage, the absence of the gap between the transparent, light-illuminating material and the imaging optical fiber bundle, and the elimination of a heat-shrinkable tube as an outer sheath, provides a fine optical fiber unit that can be easily assembled into or branched from an imaging fiber system.

**Claims**

1. A method of making an imaging catheter, characterized by the steps of:
passing an image-transmitting optical fiber bundle (5) through a first-die (15);
extruding a light-absorbing layer (13) from said first die to coat said image-transmitting optical fiber bundle with said light-absorbing layer;
passing said coated image-transmiting optical fiber bundle through a second die (16);
extruding a transparent plastic material from said second die to further coat said coated image-transmitting optical fiber bundle with an elongate body (12) of said transparent plastic material to form a composite optical fiber unit (9) of integral structure,
and preparing and end portion of said composite optical fiber unit for connection of said transparent plastic body to a light surce (1) and of said optical fiber bundle to a sensor or a direct-vision adapater (6).

2. The method as claimed in claim 1, characterized in that said step of preparing an end portion of said composite optical fiber unit for connection to a light source and a sensor or a direct-vision adapter comprises the steps of:
treating said end portion of said composite optical fiber unit with an acid to remove only said transparent material and leave said image-transmitting optical fiber bundle exposed for connection to a sensor or a direct vision adapter;
mirror-polishing a part of the exposed end-surface (19) of said transparent material to obtain a mirror-smooth surface (20);
spreading a film of matching oil on said mirror-polished surface; and
connecting an end of an auxiliary optical fiber (21) to said oil coated, mirror-polished surface for connection of its other end to a light source.

3. A method as claimed in claim 1, characterized in that said step of preparing an end portion of said composite optical fiber unit for connection to a light source and a sensor or a direct-vision adapter comprises the steps of:
splitting said end portion of·said body of transparent material into two portions along a longitudinal direction of said composite optical fiber unit;
separating said image-transmitting optical fiber bundle from said two portions of said transparent material for connection of said bundle to a sensor or a direct-vision adapter; and

joining said two portions of said transparent material for connection to a light source.

4. The method as claimed in any of claims 1 to 3, characterized by the further steps of:
forming a longitudinal opening (14) in said transparent material as said transparent material is extruded from said second die to serve as a brine conduit; and,
further preparing said end portion of said composite optical fiber unit for connection of said brine conduit to a brine-supplying syringe, by inserting an end of a tube (18) into said opening in said transparent material for connection of its other end to said syringe and by fixing said tube into said opening with an adhesive.

5. An imaging catheter made according to the method as claimed in claim 1, characerized in that it comprises:
an elongate body (12) of transparent material for transmitting illuminating light from a source (1) to a target to be viewed; and an image-transmitting optical fiber bundle (5) for transmitting an image from said target to a sensor or a direct-vision adapter (6), said image-transmitting optical fiber bundle being contained within said elongate body of transparent material, whereby said image transmitting fiber bundle (5) is coated with a light-absorbing layer (13) extruded theraround for preventing illuminating light from being leaked from said elongate body of transparent material to said image-transmitting optical fiber bundle, and whereby said elongate body of transparent material is formed of a transparent plastic material extruded around said coated image-transmitting optical fiber bundle so as to further coat it and to contain the same as an integral part thereof, thereby forming a composite unit (9) of integral structure.

6. The imaging catheter as claimed in claim 5, characterized in that said light absorbing layer consists essentially of a hard silicone resin mixed with a fine carbon carbon powder, said light-absorbing layer having a low light transmittance and a refractive index higher than that of quartz.

7. The imaging catheter as claimed in claim 5 or 6, characterized in that said transparent material is made of a material having a high light transmittance, said high light transmittance material being selected from the group consisting of polymethyl methacrylate, polystyrene, polycarbonate, and so on.

8. The imaging catheter as claimed in any of claims 5 to 6, characterized by a longitudinal liquid passage (14) formed within said body of transparent material, said liquid passage being substantailly parallel to said image-transmitting optical fiber bundle.

9. The imaging catheter as claimed in any of claims 5 to 8, characterized by a cladding layer formed on the outer surface of said body of transparent material, said cladding layer being made of a material having a lower refractive index than that of said transparent material.

10. The imaging catheter as claimed in claim 9, characterized by a protective coating located on said cladding layer.

11. The imaging catheter as claimed in claim 10, characterized in that said protective coating consists essentially of a fluorinated resin.

12. The imaging catheter as claimed in any of claims 5 to 11, characterized by a protective coating formed on a surface of said body of transparent material.

13. The imaging catheter as claimed in claim 12, characterized in that said protective coating consists essentially of a fluorinated resin.

**Patentansprüche**

1. Verfahren zur Herstellung eines Abbildungskatheders, gekennzeichnet durch die Verfahrensschritte:

Durchführen eines bildübertragenden optischen Faserbündels (5) durch eine erste Preßform (15);

Extrudieren einer Lichtabsorbierenden Schicht (13) aus der ersten Matrize, um das bildübertragende optische Faserbündel mit der lichtabsorbierenden Schicht zu überziehen;

Durchführen des überzogenen bildübertragenden optischen Fasterbündels durch eine zweite Preßform (16);

Extrudieren eines transparenten Plastikmaterials aus der zweiten Preßform, um das bildübertragende überzogene optische Faserbündel weiter mit einem längsausgedehnten Körper aus dem durchsichtigen Plastikmaterial zu ummanteln, um eine zusammengestzte optische Fasereinheit (9) von integraler Struktur zu bilden; und

Herstellen eines Endteiles der zusammengestzten optischen Fasereinheit für die Verbindung des transparenten Plastikkörpers mit einer Lichtquelle (1) und des optischen Faserbündels mit einem Sensor oder einem Direktsichtadapter (6).

2. Verfahren nach Anspruch 1, dadurch gekennzeichnet, daß der Verfahrensschritt der Herstellung eines Endteiles der zusammengestzten optischen Fasereinheit zur Verbindung mit einer Lichtquelle und mit einem Sensor oder einem Direktsichtadapter die Verfahrensschritte umfaßt:

Behandeln des Endteils der zusammengestzten optischen Fasereinheit mit einer Säure, um nur das transparente Material zu entfernen und das bildübertragende Faserbündel, das zur Verbindung mit einem Sensor oder einem Direktsichadapter enthüllt wird, übrigzulassen;

Spiegel-Polieren eines Teiles der enthüllten Endoberfläche (19) des transparenten Materials, um eine spiegelglatte Oberfläche (20) zu erhalten;

Auftragen eines Films von Kontaktöl auf die spiegelpolierte Oberfläche; und

Verbinden eines Endes einer optischen Hilfsfaser (21) mit der öl- bedeckten, spiegelpolierten Oberfläche zur Verbindung ihres anderen Endes mit einer Lichtquelle.

3. Verfahren nach Anspruch 1, dadurch gekennzeichnet, daß der Verfahrensschritt der Herstellung eines Endteiles der zusammengestezten optischen Fasereinheit für die Verbindung mit einer Lichtquelle und einem Sensor oder einem Direktsichtadapter die Verfahrensschritte umfaßt:

Aufspalten des Endteiles des Körpers aus transparentem Material in zwei Teile entlang einer Langsrichtung der zusammengestzten optischen Fasereinheit;

Trennen des bildübertragenden optischen Faserbündels von den zwei Teilen des transparenten Materials zur Verbindung des Bundles mit einem Sensor oder einem Direktischtadapter; und Verbinden der zwei Teile des transparenten Materials für die Verbindung mit einer Lichtquelle.

4. Verfahren nach einem der Ansprüche 1 bis 3, gekennzeichnet durch die weiteren Verfahrensschritte:

Bilden einer Längsöffnung (14) in dem transparenten Material, wenn das transparente Material, wenn das transparente Material aus der zweiten Preßform extrudiert wird, um als eine Solenleitung zu dienen; und

Weiterbearbeiten des Endteiles der zusammengestezten optischen Fasereinheit zur Verbindung der Solenleitung mit einer Solenzuführungsspritze, indem ein Ende einer Röhre (18) in die Öffnung im transparenten Material zur Verbindung ihres anderen Endes mit der Spritze eingeführt und die röhre in der Öffnung mit einem Kleber befestigt wird.

5. Abbildungskatheder hergestellt entsprechend dem Verfahren nach Anspruch 1, dadurch gekennzeichnet daß er umfaßt:

einen ausgedehnten Körper (12) aus einem transparenten Material zur Übertragung von illuminierendem Licht von einer Lichtquelle (1) auf ein zu beobachtendes Objekt;

und ein bildübertragendes optisches Faserbündel (5) für die Übertragung eines Bildes von dem Objekt zu einem Sensor oder einem Direktsichtadapter (6), wobei das bildübertragende optische Faserbündel in dem ausgedehnten Körper aus einem transparenten Material enthalten und das bildübertragende optische Faserbündel mit einer lichtabsorbierenden Schicht (13), die, um zu verhindern, daß illuminierendes Licht von dem ausgedehnten Körper aus transparentem Material in das bildübertragende optische Faserbündel übertritt, um das bildübertragende Bündel herum extrudiert ist, und wobei der ausgedehnte Körper aus transparentem Material aus einem transparentem Plastikmaterial, das um das überzogene bildübertragende optische Faserbündel herum extrudiert ist, so daß, um es weiter zu ummanteln und es als integralen Bestandteil davon zu umfassen, wobei eine zusammengesetzte Einheit (9) von integraler Struktur gebilden wird.

6. Abbildungskatheder nach Anspruch 5, dadurch gekennzeichnet, daß die lichtabsorbierende Schicht im wesentlichen aus einem harten Silikonharz gemischt mit einem feinen Kohlenstoffpulver besteht, wobei die lichtabsorbierende Schicht eine geringe Lichtübertragungsfähigkeit und einen Brechungsindex größer als der Brechungsindex von Quarz aufweist.

7. Abbildungskatheder nach Anspruch 5 oder 6, dadurch gekennzeichnet, daß das transparente Material ein Material mit einer hohen Lichtübertragungsfähigkeit ist, wobei das Material mit einer hohen Lichtübertragungsfähigkeit aus der aus Polymethylmetacrylat, Polysterol, Polycarbonat, usw. bestehenden Gruppe ausgewählt ist.

8. Abbildungskatheder nach einem der Ansprüche 5 bis 7, gekennzeichnet durch einen längs ausgedehnten Flüssigkeitsdurchgang (14), der innerhalb des Körpers aus transparentem Material gebildet ist, wobei der Flüssigkeitsdurchgang im wesentlichen parallel zu dem lichtübertragenden Faserbündel vorgesehen ist.

9. Abbildungskatheder nach einem der Ansprcüche 5 bis 8, gekennzeichnet durch eine bedeckende Schicht, die auf der äußeren Oberfläche des Körpers aus transparentem Material gebildet ist, wobei die Deckschicht aus einem Material mit einem geringeren Brechungsindex als der Brechungsindex des transparenten Materials hergestellt ist.

10. Abbildungskatheder nach Anspruch 9, gekennzeichnet durch einen Schutzüberzug, der auf der Deckschicht angeordnet ist.

11. Abbildungskatheder anch Anspruch 10, dadurch gekennzeichnet, daß der Schutzüberzug im wesentlichen aus einem fluorierten Harz besteht.

12. Abbildungskatheder nach einem der Ansprüche 5 bis 11, gekennziechnet durch eine Schutzschicht, die auf einer Oberfläche des Körpers aus transparentem Material gebildet ist.

13. Abbildungskatheder nach Anspruch 12, dadurch gekennzeichnet, daß die Schutzschicht im wesentlichen aus einem fluorierten Harz besteht.

## Revendications

1. Procédé de fabrication d'un cathéter transmetteur d'images, caractérisé par les étapes suivantes:

passage d'un faisceau de fibres optiques de transmission d'images (5) à travers une permière filière (15);

extrusion d'une couche absorbant la lumière (13) à partir de ladite permière filière pour recouvrir ledit faisceau de fibres optiques de transmission d'images de ladite couche absorbant la lumière;

passage dudit faisceau de fibres optiques de transission d'images revêtu à travers une seconde filière (16);

extrusion d'une matière plastique transparente à partir de ladite second filière pour recouvrir encore ledit faisceau de fibres optiques de transmission d'images revêtu d'un corps allongé (12) en ladite matière plastique transparente pour former une unité de fibres optiques composite (9) de structure monobloc, et

préparation d'une partie extrême de ladite unité de fibres optiques composite pour relier ledit corps en matière platique transparente à une source lumineuse (1) et ledit faisceau de fibres optiques à un capteur ou à un adaptateur de vision directe (6).

2. Procédé selon la revendication 1, caractérisé en ce que ladite étape de préparation d'une partie extrême de ladite unité de fibres optiques composite pour une liaison à une source lumineuse et à capteur ou à un adaptateur de vision directe comprend les étapes suivantes:

traiter ladite partie extrême de ladite unité de fibres optiques composite par un acide pour éliminer seulement ladite matière transparente et pour laisser ledit faisceau de fibres optiques de transmission d'images exposé en vue d'une liaison à un capteur ou à un adaptateur de vision directe;

polir à reflets une partie de la surface extrême (19) exposée de ladite matiére transparente pour obtenir une surface (20) polie-miroir;

étaler un film d'huile a'adaptation sur ladite surface polie-miroir; et

relier une extrémité d'une fibre optique auxiliare (21) à ladite surface polie-miroir revêtue d'huile pour une liaison de son autre extrémité à une source lumineuse.

3. Procédé selon la revendication 1, caractérisé en ce que ladite etape de préparation d'une partie extrême de ladite unité de fibres optiques composite pour une liaison à une source lumineuse et à un capteur ou à un adaptateur de vision directe comprend les étapes suivantes:

diviser ladite partie extrême corps de matière transparente en deux parties suivant une direction longitudinale de ladite unité de fibres optiques composite;

séparer ledit faisceau de fibres optiques de transmission d'images depuis lesdites deux parties de ladite matière transparente pour une liaison dudit faisceau à un capteur ou à un adapteur de vision directe; et

joindre lesdites deux parties de ladite matière transparente pour une liaison à une source lumineuse.

4. Procédé selon l'une des revendications 1 à 3, caractérisé par les étapes supplémentaires suivantes:

former une ouverture longitudinale (14) dans ladite matière transparente lorsque ladite matière transparente est extrudée à partir de ladite seconde filière pour servir de conduite d'eau salée; et

préparer encore ladite partie extrême de ladite unité de fibres optiques composite pour une liaison de ladite conduite d'eau salée avec une seringue de fourniture d'eau salée, par insertion d'une extrémité d'une tube (18) dans ladite ouverture de ladite matière transparente pour une liaison de son autre ladite ouverture avec un adhésif.

5. Cathéter transmetteur d'images fabriqué selon le procédé de la revendication 1, caractérisé en ce qu'il comprend.

un corps allongé (12) en matière transparente pour transmettre la lumière d'éclairage provenant d'une source lumineuse (1) à une cible à observer; et

un faisceau de fibres optiques de transmission d'images (5) pour transmettre une image provenant de ladite cible à un capteur ou adaptateur de vision directe (6), ledit faisceau de fibres optiques de transmission d'images étant contenu à l'intérieure dudit corps allongé de matière transparente, de sorte que ledit faisceau de fibres optiques de transmission d'images (5) est recouvert d'une couche absorbant la lumière (13) extrudée autour de celui-ci pour empêcher la lumière d'élairage de s'échapper dudit corps allogne de matière transparente vers ledit faisceau de fibres optiques de transmission d'images, et de sorte que ledit corps allongé de matière transparente est constitué par une matière plastique transparente extrudée autour dudit faisceau de fibres optiques de transmission d'images revêtu de façon à le recouvrir encore et à la contenir comme partie intégrante de celui-ci, formant ainsi une unité composite (9) de structure monobloc.

6. Cathéter transmetteur d'images selon la revendication 5, caractérisé en ce que ladite couche absorbant la lumière consiste essentiellement en une résine de silicone dure mélangée à une fine poudre de carbone, ladite couche absorbant la lumière ayant un faible facteur de transmission de la lumiére et un indice de réfraction supérieur à celui de quartz.

7. Cathéter transmetteur d'images selon la revendication 5 ou 6, caractérisé en ce que ladite matière transparente est en une matière ayant un facteur de transmission de la lumière élevé, ladite matière à facteur de transmission élevé étant choisie dans le groupe consistant en le polymé-thylnéthacrylate, le polystyrène, le polycarbonate, etc.

8. Cathéter transmetteur d'images selon l'une des revendications 5 à 7, caractérisé par un passage de liquide longitudinal (14) formé à l'intérieur dudit corps de matière transparente, ledit passage de luquide étant sensiblement parallèle audit faisceau de fibres optiques de transmission d'images.

9. Cathéter transmetteur d'images selon l'une des revendications 5 à 8, caractérisé par une couche de placage formée sur la surface externe dudit corps en matière transparente, ladite couche de placage étant en une matière ayant un indice de réfraction inférieure à celui de ladite matière transparente.

10. Cathéter transmetteur d'images selon la revendication 9, caractérisé par un revêtement protecteur situé sur ladite couche de placage.

11. Cathéter transmetteur d'images selon la revendication 10, caractérisé en ce que ladit revêtement protecteur consiste essentiellement en une résine fluorée.

12. Cathéter transmetteur d'images selon l'une des revendications 5 à 11, caractériséd par un revêtement protecteur formé sur une surface dudit corps en matiére transparente.

13. Cathéter transmetteur d'images selon la revendication 12, caractérisé en ce que ledit revêtement protecteur consiste essentiellement en une résine fluorée.

FIG. 1 PRIOR ART

FIG. 2
PRIOR ART

FIG. 3

FIG. 4

FIG. 5

FIG. 6

FIG. 7

FIG. 8

FIG. 9